# EUROPEAN PATENT SPECIFICATION

(11) **EP 2 506 016 B1**
(45) Date of publication and mention of the grant of the patent: **08.07.2015**
(21) Application number: 10831836.1
(22) Date of filing: 23.11.2010
(51) Int. Cl.: G01N 33/68

(54) **DIAGNOSTIC METHOD FOR DETECTING ACUTE KIDNEY INJURY USING HEAT SHOCK PROTEIN 72 AS A SENSITIVE BIOMARKER**
DIAGNOSTISCHES VERFAHREN FÜR DEN NACHWEIS VON AKUTER NIERENLÄSION MIT DEM HITZESCHOCKPROTEIN 72 ALS SENSITIVER BIOMARKER
MÉTHODE DE DIAGNOSTIC POUR DÉTECTER UNE LÉSION RÉNALE AIGUË GRÂCE À L'UTILISATION DE LA PROTÉINE DE CHOC THERMIQUE DE 72KDA EN TANT QUE BIOMARQUEUR SENSIBLE

(30) Priority: 23.11.2009 MX MX09012633
(43) Date of publication of application: 03.10.2012
(73) Proprietor: Universidad Nacional Autónoma De México, México, D.F. C.P. 04510 (MX)
(72) Inventor: BOBADILLA SANDOVAL, Norma Araceli, C.P. 09800 México D.F. (MX); BARRERA CHIMAL, Jonatan, C.P. 55430 Ecatepec Estado de México (MX)
(74) Representative: Carvajal y Urquijo, Isabel
(86) International application number: PCT/MX2010/000138
(87) International publication number: WO 2011/062469

(56) References cited:
- BARRERA-CHIMAL JONATAN ET AL: "Hsp72 is an early and sensitive biomarker to detect acute kidney injury", EMBO MOLECULAR MEDICINE, vol. 3, no. 1, January 2011 (2011-01), pages 5-20, XP002688968,
- MUELLER, T. ET AL.: 'Urinary heat shock protein-72 excretion in clinical and experimental renal ischemia' PEDIATRIC NEPHROLOGY. vol. 18, no. 2, 2003, pages 97 - 99, XP008156569
- ZHANG, P.L. ET AL.: 'Heat shock protein expression is highly sensitive to ischemia-reperfusion injury in rat kidneys' ANNALS OF CLINICAL & LABORATORY SCIENCE. vol. 38, no. 1, 2008, pages 57 - 63, XP002654875
- VAN WHY, S.K. ET AL.: 'Induction and intracellular localization of HSP-72 after renal ischemia' AM. J. PHYSIOL. RENAL PHYSIOL. vol. 263, 01 November 1992, pages F769 - F775, XP008156578

## Description

### FIELD OF THE INVENTION

The present invention fits into the clinical medicine area and refers to a diagnostic method for detecting Acute Kidney Injury (AKI), more specifically refers to the demonstration that the heat shock protein of 72 kDa (Hsp72) is a non-invasive, sensible and early biomarker to detect AKI and to the quantification methods for detecting Hsp72 in urine samples.

### BACKGROUND OF THE INVENTION

Acute kidney injury is an important cause of morbidity and mortality among hospitalized patients for different causes. It is estimated that AKI incidence diverges from 5% in patients with normal renal function before any surgery to a 30% in patients admitted to the intensive care unit (ICU). In spite of recent advances in the diagnosis and therapeutics, the AKI associated morbidity and mortality remains highly elevated (40% to 60% in patients in the ICU) and has not been considerably improved in the past four decades, mainly due to lack of sensitivity and specificity of the available tools for early detection of AKI (Clin J Am Soc Nephrol 3:1895-1901,2088). Because of this, the search for early biomarkers is gaining great importance. Moreover, these new biomarkers might be potential tools for early detection of AKI and might be also able to distinguish different degrees of kidney injury in order to establish detect those patients that are in risk to develop chronic kidney disease, due to a severe AKI episode. Therefore, the development of effective biomarkers will help to make an opportune intervention for an adequate treatment of AKI, in those patients exposed to AKI such as; patients admitted to the ICU, those that will be subjected to cardiac surgery, renal transplant patients or those that have developed AKI and the biomarker will help to stratify the injury, as well as to detect those patients that are at risk of chronic kidney disease development.

In the clinical practice the diagnosis of AKI is established based on elevations of serum creatinine and by estimations of the glomerular filtration rate (GFR). Though serum creatinine is useful for renal function estimation in chronic kidney disease patients, in AKI patients, it is not a good indicator for following three reasons: 1) A great amount of the renal tissue may be injured without serum creatinine elevations, a clear example occurs in renal transplant donors, whom loose 50% of the kidney mass and do not present any changes in the serum creatinine levels, 2) serum creatinine concentration depends on many non-renal factors such as; conversion of creatine to creatinine in the skeletal muscle, creatinine liberation into the blood, etc., so the elevation in serum creatinine happens in a late fashion as depends on its liberation and accumulation and 3) serum creatinine may be influenced by other factors such as; weight, race, gender, age, drug consumption, muscular metabolism and protein intake. Respect to GFR determination, this can be modified for renal and non-renal injuries. For example; hypovolemia or alterations in the degree of vasoconstriction or vasodilation in the afferent arteriole cause a reduction in TGF with a consequent elevation in serum creatinine, which is not correlated with tubular or renal injury. All these factors difficult the early intervention of the patients that develop AKI and in consequence a better prognosis is not reached. (Clin Transl Sci 3;200-208;2008).

A biomarker is a biologic molecule that is endogenously produced and that may be an objective indicator for detecting an abnormal biological process. Furthermore it can help to detect if a pharmacological intervention is being useful for reducing the injury provoked by the pathologic process.

Specifically, in AKI patients, a useful biomarker will be the one that helps to detect in an early, precise and easy fashion the main structural complication of AKI that is acute tubular necrosis (ATN). ATN is characterized by severe proximal tubule injury due to the loss of the brush border and polarity in the epithelium. For these characteristics, its possible to find a biomarker that can be detected in the detached cells and that will appear in the urine, thus reflecting the tubular injury associated to this syndrome.

The biomarkers will not just help to differentiate ATN from other types of renal injury, but also may potentially identify the tubular injury localization, the cause and the temporal curse of the injury.

Several studies have proposed proteins and biochemical markers for ATN detection, among them are: N-acetil-b-D-glucosaminidasa (NAG), Neutrophil gelatinase associated lipocalin (NGAL), kidney injury molecule-1 (Kim-1), Cystatin C and interleukin-18 (IL-18) (Am J Physiol Renal Physiol 290:F517-F529) (J Am Soc Nephrol 18:904-912, 2007) (Am J of Tranplantation 6:1639-1645;2006). In a recent report with 90 patients subjected to cardiac surgery, the diagnostic utility of Kim-1, NAG and NGAL was evaluated. By analyzing the ability of these molecules for AKI detection in an immediate fashion or 3 hours after the cardiac surgery, using a scale of 1, it was found that Kim-1 capacity was 0.68 and 0.65 respectively, for NAG: 0.61 and 0.63 and for NGAL: 0.59 and 0.65). To increase the sensibility of these markers it was necessary to combine the quantification of them, and in this way the sensibility and the early detection of AKI was increased to 0.75 and 0.78, respectively (Clin J Am Soc Nephrol 5;873-882,2009). This support the idea that the search for novel biomarkers with a higher potential for early diagnosis and with the ability to stratify the AKI injury must be continued.

It is reported that during AKI phenomena, several mechanisms to compensate the resultant cell stress are activated, one of them is the increase in the expression of the heat shock protein family (Hsp) (Experientia 18, 571-573,1962) that help to restore the cell homeostasis. These proteins belong to a multigenic family with a molecular weight that goes from 10 to 150 kDa. These proteins are classified according to their molecular weight in 6 subfamilies: 100-110 kDa, 90 kDa, 70 kDa, 60 kDa, 40 kDa and the Hsp subfamily with molecular weight between 18 and 30 kDa (Ann Med. 4:261-71,1999).

In particular the family of the Hsp70 is compose by 4 isoforms; Grp78, mHsp95, Hsc70 and the inducible isoform: Hsp72. The later is expressed after cell stress and its induction can become as high as 15% of the total cell protein (Cell stress chaperones 4;309-316,2003). This fact, together with the cell detachment from the proximal tubule of the nephron that occurs during AKI, was used as the base for our invention that is: urinary Hsp72 detection, as a sensitive biomarker for AKI, at both the protein level, using immunoassays and at the mRNA level using the real time-polymerase chain reaction (real time PCR).

Müller discloses the detection of Hsp70/72 in the urine of transplanted children and rats subjected to ischemia (Müller T et al. "Urinary Heat Shock Protein-72 Excretion in Clinical and Experimental Renal Ischemia", Pediatric Nephrology, 2003, 18, 2, 97-99). However, it does not demonstrate renal injury in the transplanted children. The possible kidney damage should have been assessed either by increased serum creatinine or histopathologic evaluation, which are missing in the publication. Similar situation happens with the experimental animals: the assays were conducted in rats subjected to ischemia, not showing kidney damage either. The document does not assess that the presence of Hsp72 in the urine reflects any degree of kidney functional or structural abnormality, contrary than the present invention. Indeed, from the transplanted children only the urine results of three of them are referred to, and these without specifying what kind of donor they are, whether living or cadaveric donor. This fails to support serious considerations towards an increased level of Hsp72 indicative of any abnormality, known that in the short term after kidney transplantation the immune system activates Hsp72 expression.

Zhang shows that during a process of kidney ischemia / reperfusion in the rat, an increase of Hsp70 and Hsp27 levels happen in the renal tissue (Zhang PL. et al, "Heat Shock Protein Expression is Highly Sensitive to Ischemia-Reperfusion Injury in Rat Kidneys", Annals of Clinical & Laboratory Science, 2008, 38, 1, 57-63). This publication recognizes both of them as different proteins, although unfortunately does not disclose the used antibody for identification. It is important to note that the behaviour of Hsc70 after ischemia is very different than the induction of Hsp72. Indeed, Zhang does not mention that the concentration of Hsp72 rises, which detection in the urine of the tested animals would have been even less probable. It is important to note that the induction of heat shock proteins in ischemic conditions it is a well-known phenomenon in the art. This is not restricted to Hsp72, as it happens with other heat shock proteins at else other organs. The function of these proteins is inducing themselves to restore cellular homeostasis.

Van Why discloses an increase in the levels of mRNA and protein of Hsp72 in the whole rat kidney subjected to ischemia (Van Why SK, et al. "Induction and Intracellular Localization of HSP-72 after Renal Ischemia", AM, J. Physiolo. Renal Physiol., 1992, 263, F769-F775). The said induction in the kidney tissue falls within the provisions of above. In addition, Hsp72 is abundantly expressed in the renal medulla constitutively, for Van Why to measure it; different than in renal cortex wherein it is induced under acute renal injury to be measured in the urine as per the present invention.

ELISA (Enzyme linked immunosorbent assay) technique and Western blot analysis have been widely used for specific protein detection with the use of antibodies, in different types of samples (Immunology 6th edition, 2007).

The real time PCR test is used for quantitative determination of mRNA levels of a specific gene.

This invention contributes to solve the problem that exist in the clinical practice of being unable for early detection of AKI and to stratify the degree of renal injury that the kidney suffered, in order to make an opportune intervention of the patients with an effective therapy.

### DESCRIPTION OF THE INVENTION

The present invention relates to a non-invasive diagnostic method to early detection of acute kidney injury by using the concentration of the biomarker Hsp72 in urine samples. This is a non-invasive, trustable and easy method.

In the present invention, the method for detecting AKI goes from obtaining a urine sample from a mammal, preferentially humans and the quantification of the biomarker concentration; heat shock protein 72 (Hsp72), at both the protein and the mRNA level.

The biomarker's concentration may be determined at the mRNA level and/or protein level using immunoassays, such as ELISA and western blot analysis, without this limiting the invention.

The result from the biomarker quantification varies between 40 and 533 fold compared to control values and this increase depends from the injury intensity and the biomarker Hsp72 in the urine can be detected since the three hours after the injury has been provoked in the kidney.

Hsp72 quantification is able to stratify the intensity of the injury provoked by increasing periods of ischemia, which is important in the clinical practice in order to detect those patients that suffered from severe renal injury, which in turn will allow an opportune follow-up and in consequence it could be of great impact because it can avoid or reduce the chronic kidney disease complication.

### EXAMPLES

The following examples are to illustrate the invention and in any case to limit it.

Example 1. To demonstrate Hsp72 usefulness as a sensible and early biomarker of AKI, we used the renal ischemia/reperfusion (I/R) model in the rat. *Ischemia*/*reperfusion model:* Male Wistar rats were used throughout the study. The rats were anesthetized with sodium pentobarbital (30 mg/kg i.p.), laparotomy was performed and the renal pedicles were dissected, thereafter the blood flow was interrupted to the kidneys by clamping both arteries during 10, 20, 30, 45 and 60 minutes with the objective of evaluating different degrees of renal injury, since low injury to moderate and severe kidney injury. Furthermore, a group subjected to false surgery was included as a control. Each group was conformed for 6 rats. At the end of the ischemia period, the rats were sutured and the renal reperfusion was allowed for 24 hr. To determine the utility of quantifying Hsp72 mRNA levels as a biomarker, 36 rats divided in 6 groups were used; control group and the rats subjected to bilateral ischemia of 10, 20, 30, 45 and 60 min, all of them with 24 h of reperfusion. The urine was collected with special conditions to avoid mRNA degradation as is described later.

In a similar way and for determining the usefulness of the quantification of the protein Hsp72 levels as an early biomarker, 33 rats more were divided in 11 groups; control group with sham surgery and the rats with bilateral ischemia of 30 min and reperfusion periods of 3, 6, 9, 12, 18, 24, 48, 72, 96 and 120 h. The urine was collected to ascertain the sensibility of Hsp72 protein levels as an early biomarker of AKI using an ELISA method.

In all the groups, renal function was assessed by creatinine clearance and by measuring renal blood flow. Structural injury was evaluated using light microscopy and morphometry. As tubular injury markers, urinary NAG and total protein levels were measured. To study if Hsp72 is induced during ischemia in the kidney, mRNA and protein levels of Hsp72 were evaluated in the kidney tissue extracts. To determine if Hsp72 is a sensible and early biomarker to detect different degrees of renal injury, the urinary Hsp72 levels were quantified using ELISA and Western blot.

### EXAMPLE 2. Hsp72 detection by using real time PCR.

For Hsp72 mRNA levels detection, 30 male Wistar rats were subjected to bilateral ischemia of 30 min were divided into six groups: rats subjected to sham surgery (control group) and rats subjected to bilateral ischemia of 10, 20, 30, 45 and 60 min and 24 of reperfusion. One hour after the surgery the rats were housed into metabolic cages for 24 hours. The metabolic cages were previously treated with an RNA inhibitor (RNAse Zap, Ambion). In the tube, where the urine was collected for 24 h, 300 µl of RNA later (Ambion) were added and the samples were centrifuged at 3000 rpm during 30 min. The urinary sediment was resuspended in phosphate buffer pH=7.4 and was again centrifuged at 13000 rpm during 3 min. The total RNA extraction was made according to the Trizol method given by the manufacturer (Invitrogen). RNA concentration was determined by UV absorbance at 260 nm and RNA integrity was corroborated by 1% agarose gel electrophoresis. Each cDNA was synthesized from 1 µg of RNA using a reverse transcriptase reaction (RT) at 37 C during 60 min. Hsp72 mRNA levels were detected by real time PCR. Ribosomal RNA 18S was included as a control gene to correct the amplification efficiency variations.

### EXAMPLE 3. Hsp72 detection by using ELISA.

For Hsp72 protein levels detection, 36 rats were included and subjected to bilateral ischemia of 10, 20, 30, 45 and 60 min. One hour after the surgery, the rats were putted in the metabolic cages for 24 hours and the urine was collected. The urine must be used immediately for ELISA or western blot assays, otherwise must be stored at -80 C to avoid Hsp72 degradation. For Hsp72 quantification by ELISA the commercial kit Hsp70 High sensitivity ELISA kit produced by Stressgene was used as is briefly explained:
1) 100 µl of the urine samples was added to each well of the ELISA plate;
2) The plate was incubated for 2 h at room temperature and with gentle shaking;
3) Three washes must be performed in each well with the wash buffer provided by the kit;
4) 100 µl of the primary antibody (anti-Hsp72) must be added to the wells and the plate is incubated for 60 min. At the end of the period, three washes must be done as mentioned on point number three;
5) 100 µl of the secondary antibody coupled to HRP must be added, and was again incubated at room temperature for 60 min. At the end of the this time, three more washes must be done;
6) 100 µl of the substrate solution (from the kit) must be added to each well and the plate must be incubated for 30 min at room temperature;
7) 100 µl of the stop solution (from the kit) must be added;
8) The plate must be read at 450 nm as is stated in the manufacturer's instructions.

### EXAMPLE 4. Hsp72 detection using western blot.

The same urine samples from the rats described in the last argument were used. Each urine must be diluted 1:100 and only 10 µl of the diluted urine are used. The diluted urine was mixed with 10 µl of loading buffer (6% SDS, 15% glycerol, 150 mM Tris, 3% bromophenol blue, 2% β-mercaptoetanol, pH 7.6). The proteins were denatured at 95 C for 5 min, electrophoretically separated in an 8.5% SDS-PAGE gel and electroblotted to a polivinil difluoride membrane (PVDF, Amersham Pharmacia Biotech, Psicataway, NJ, USA), previously equilibrated in 1X transference buffer (190mM glycine, 2mM Tris base, SDS 0.1%, 200 mL methanol in a trans-blot (SD cell, BioRad) during 60 min at 9V and are blocked in TBS-T (Tris Buffered Saline and tween) with 5% blocking reagent (BIORAD) at room temperature. After the blocking step, the membranes were incubated overnight at 4 C, with the anti-Hsp72 primary antibody 1:5000 (Stressgene). After the incubation the membranes were washed three times 10 min each with TBS-T. Afterwards the igG goat-antimouse secondary antibody were incubated with the membranes 1:5000 (Santa Cruz Biotechnology Inc) during 90 min at room temperature, and the membranes were washed again for 6 times. The Hsp72 amount was detected using the commercially available kit ECL plus (GE Healthcare Life Sciences) and the obtained bands were scanned for densitometry analysis.

### RESULTS

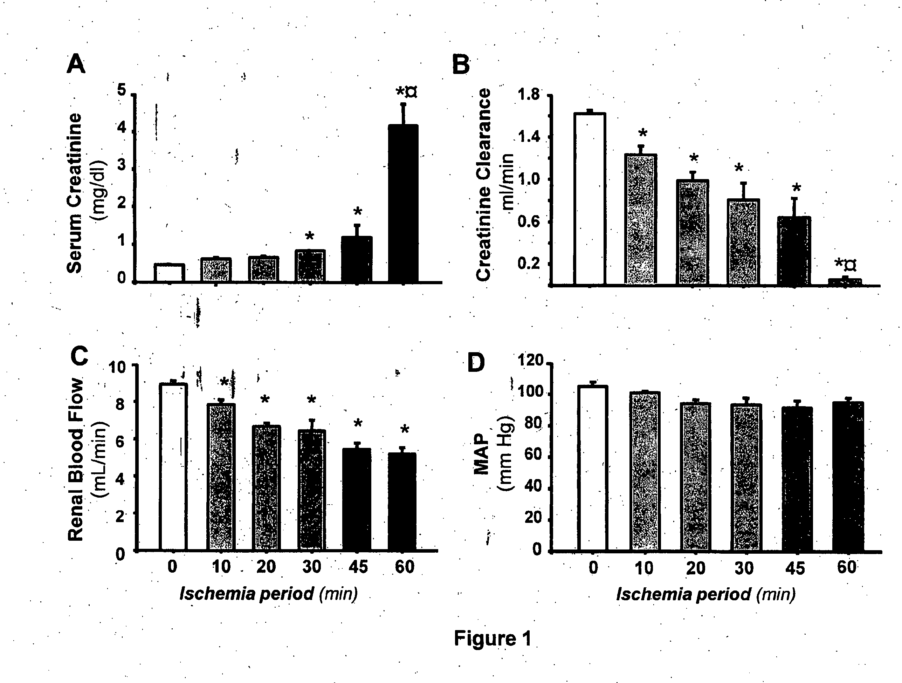

First, we evaluated the effect of producing different periods of bilateral ischemia (10 to 60 min) and 24 of reperfusion over the renal function. The five groups of rats subjected to ischemia, developed renal dysfunction, evidenced by a progressive elevation in serum creatinine and a reduction in the GFR measured by the creatinine clearance as is shown in Figures 1A and 1B. Renal dysfunction was associated with a reduction from 10 to 30% in the renal blood flow, without changes in the mean arterial pressure as is detailed in Figures 1C and 1D.

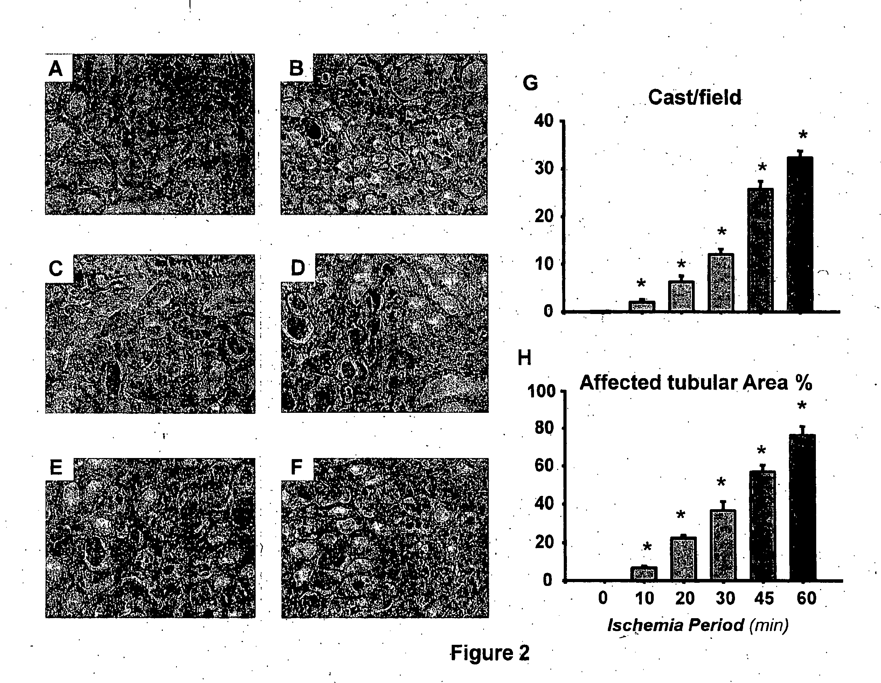

Light microscopy studies revealed that the different bilateral ischemia periods and 24 of reperfusion induced different degrees of tubular injury, according to the time of ischemia provoked, as is shown in the representative images from each group (A) to (F) from figure 2. The injury was characterized by loss of the brush border, tubular dilation, cell detachment and cast formation. The cast number per field and the percentage of the affected tubular area analysis that are shown in Figures 2G and 2H, revealed that the longer the period of ischemia, the greater the degree of tubular injury developed, which means that there was a progressive increase in the tubular injury and cast number that was proportional to the intensity of the insult. Histological injury evaluated by light microscopy is the gold standard to determine the degree of injury induced by ischemia/reperfusion, that is why in the next figures we will show the correlation between the Hsp72 biomarker and the tubular injury.

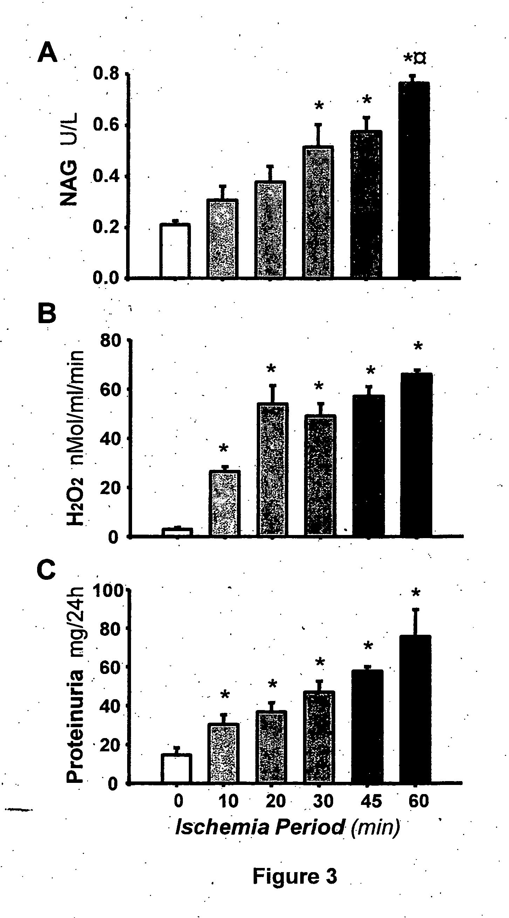

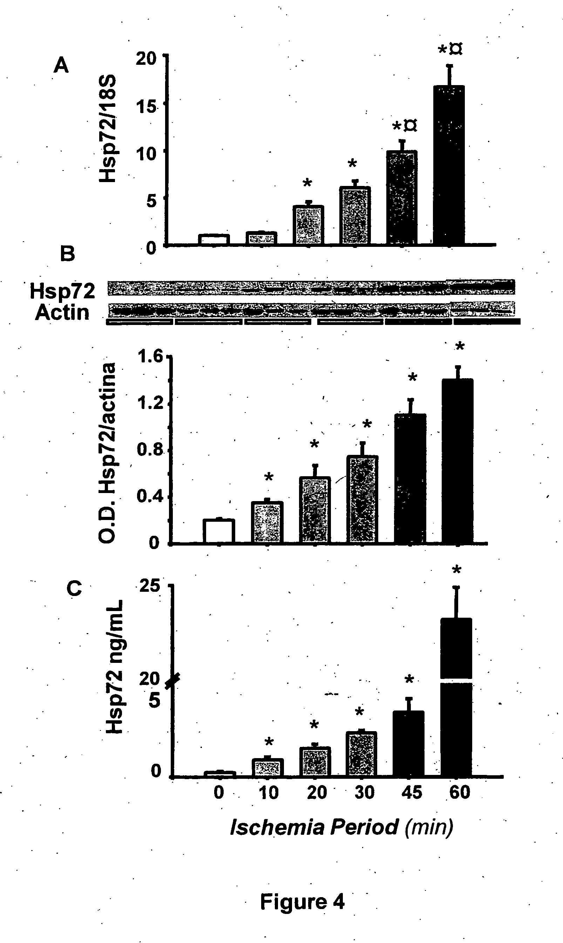

Some of the classical markers of tubular injury and oxidative stress were evaluated such as urinary protein excretion, N-acetyl-beta-D-glucosaminidase (NAG) and H₂O₂. As depicted in Figure 3A, NAG elevation was statistically significant only after 30 min of ischemia, which means that this marker was unable of identifying the renal injury induced by a lower period of ischemia (10 or 20 min). Respect to the oxidative stress marker, the H₂O₂ urinary excretion was elevated since 10 min of ischemia, but was not enough sensitive to differentiate several degrees of ischemia, especially between 20 and 60 min of ischemia, Figure 3B. Finally, we observed that the proteinuria was the best marker for detecting different degrees of renal injury as is depicted in Figure 3C. To evaluate if Hsp72 is induced in the renal tissue during different periods of ischemia, the Hsp72 mRNA and protein levels were determined in the renal tissue, it means that once the urine was collected the rats were sacrificed and one of the kidneys was obtained for mRNA and protein extraction. As is shown in Figure 4A and 4B, both the Hsp72 mRNA and protein levels were significantly increased in the renal tissue from each rat of the groups with I/R. Also, it is appreciated a progressive increase that clearly limits the different degrees of renal injury induced by different periods of ischemia/reperfusion. These results show that during an I/R phenomenon there is an increase in the Hsp72 expression, that interestingly and convenient to our invention is proportional to the degree of injury induced.

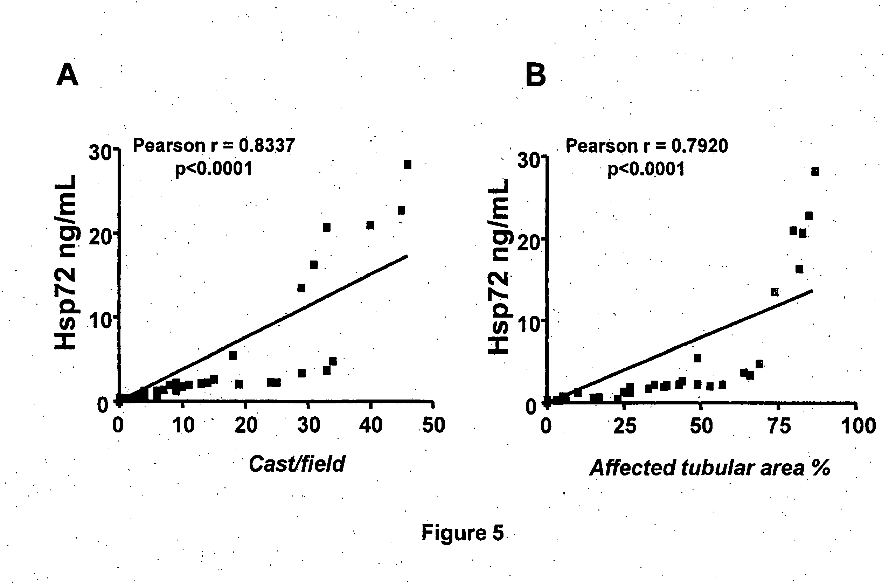

With these data we ruled out to investigate if this protein could be detected in the urine of the rats that suffered renal ischemia/reperfusion injury, to develop a sensitive and non-invasive method. For this purpose we determined the Hsp72 levels by using two immunoassays. The first was by ELISA and the second by Western blot analysis. The urinary Hsp72 quantification by ELISA revealed that this protein is an excellent biomarker from AKI, since it can be detected in the urine since 10 min of ischemia, and shows a progressively increase according to the degree of renal injury induced by ischemia, reaching a 25 fold induction in the rats subjected to 60 min of ischemia compared to the control as is shown in Figure 4C.The progressive elevation of these biomarker significantly correlated with the gold standard for ischemic injury; the histopathological analysis. Figure 5A shows the positive correlation between the amount of Hsp72 in the urine and the cast formation with a relationship of 0.83 and p<0.0001 and Figure 5B shows the correlation between urinary Hsp72 and the percentage of affected area, being 0.79 and p<0.0001.

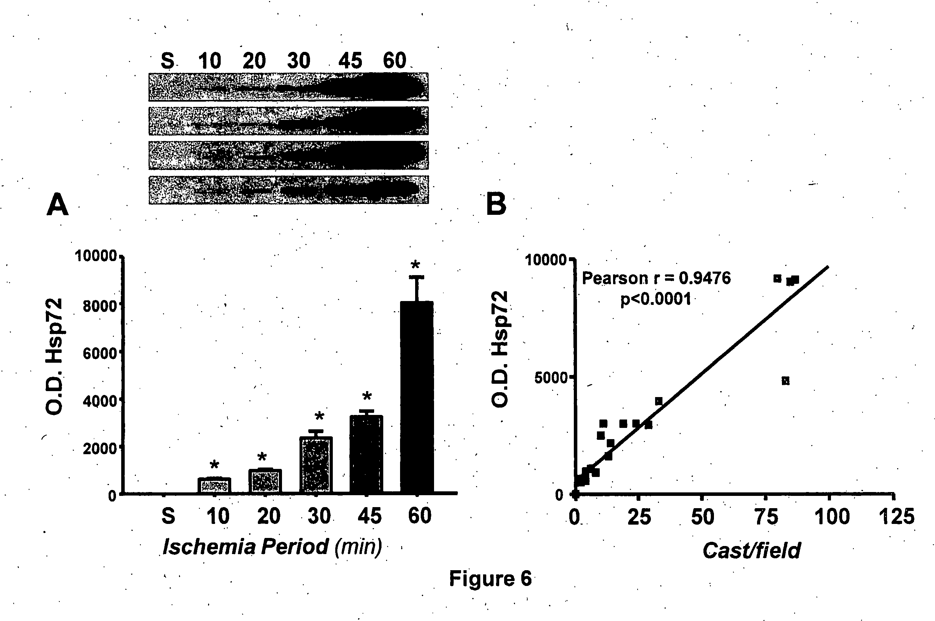

The other strategy that we used to detect the hsp72 protein levels was by Western blot analysis from the urine samples of the different groups. The upper picture in Figure 6A depicts the autoradiography from the Western blot analysis and the lower graph the densitometric analysis of the scanned bands. As it can be observed, I/R induced a significant increase in the urinary Hsp72 levels in the rats that were subjected to different periods of ischemia. Similar to the ELISA analysis, Hsp72 detection was statistically significant since 10 min of ischemia and it was elevated proportionally to the degree of renal injury induced. The sensibility to detect different degrees of renal injury was greater with the Western blot than with the ELISA, as the Hsp72 increase in the group of 10 min was 40 fold, with a progressive increase, reaching 535 fold in the group with severe renal injury of 60 min. The correlation between the amount of Hsp72 in the urine and cast formation is shown in Figure 6B, being this of 0.9476, p<0.0001. These results demonstrate that urinary Hsp72 detection is sensitive enough to detect different degrees of renal injury, however the detection of this protein by Western blot analysis was superior respect to the ELISA analysis (see correlations). Furthermore it is important to emphasize that for Western blot analysis, it is only needed 0.1 µl of the urine, while for the ELISA 100 µl are required.

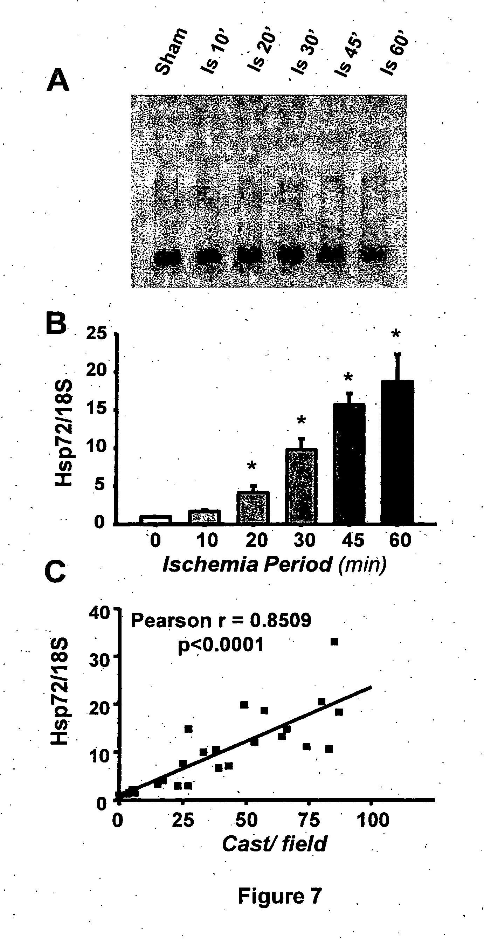

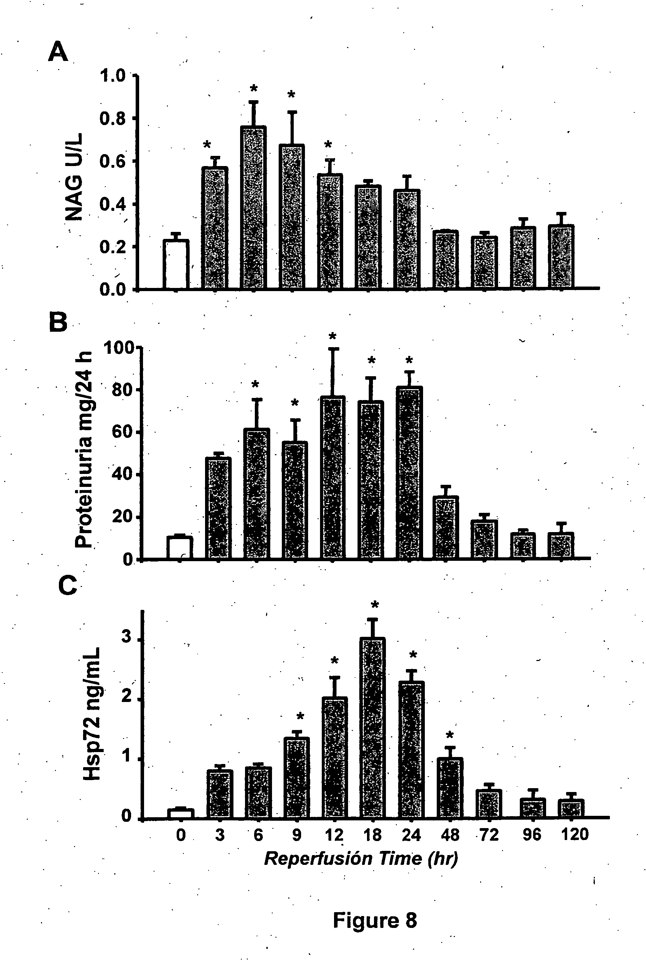

To explore if Hsp72 detection is not only limited to its quantification at the protein level, we decided to explore the Hsp72 mRNA abundance in the urine of rats underwent to ischemia. The integrity of the extracted RNA is shown in Figure 7A. The urinary levels of Hsp72 mRNA were increased in the rats subjected to ischemia respect to the control group as is depicted in Figure 7B. As in the protein levels happened, the mRNA levels in the urine proportionally increased to the degree of injury induced. This was corroborated with the gold standard and as shown in Figure 7C, there was a significant correlation of 0.8509 between the Hsp72 mRNA levels and the number of casts per field. Finally, to evaluate the Hsp72 usefulness as an early biomarker of AKI the urinary Hsp72 concentration was determined in rats subjected to 30 min of ischemia and reperfusion periods form 3 to 120 hours. Figure 8 shows Hsp72 detection, compared to other markers of tubular injury. As depicted in Figure 8C, a significant elevation of Hsp72 was observed since an early period (3 h of reperfusion), reaching a peak at 18 h of reperfusion with a later reduction in the urinary excretion of this protein, which correlates with tubular regeneration after 72 hours. These results show the utility of Hsp72 as an early biomarker for AKI detection.

Furthermore, the result form the biomarker quantification was bigger than in the control group and the increase observed is dependent on the injury intensity, result that is observed using the three different methodologies. It is important to highlight that urinary Hsp72 can be detected since the first three hours after the renal insult has been induced.

### EXAMPLE 5. Urinary Hsp72 levels in healthy living kidney donors and in AKI patients.

Samples from five healthy kidney donors were collected (controls) and from 9 patients with septic AKI from the ICU at Instituto Nacional de Ciencias Médicas y Nutrición, Salvador Zubirán. The AKI diagnosis was defined by an increase of 0.3 mg/dl or more in the serum creatinine respect to the basal, accordingly to the AKIN (Acute Kidney Injury Network) guidelines. In the healthy donors, the first urine of the day was collected one day previous to the nephrectomy (informed consent). All the sepsis patients were monitored daily and when the AKI diagnosis was established, fresh urine was collected by draining the urine collection bag. All the samples were frozen and stored at -80 C until Hsp72 levels were analyzed.

Hsp72 urinary levels in healthy donors and AKI patients. (Results).

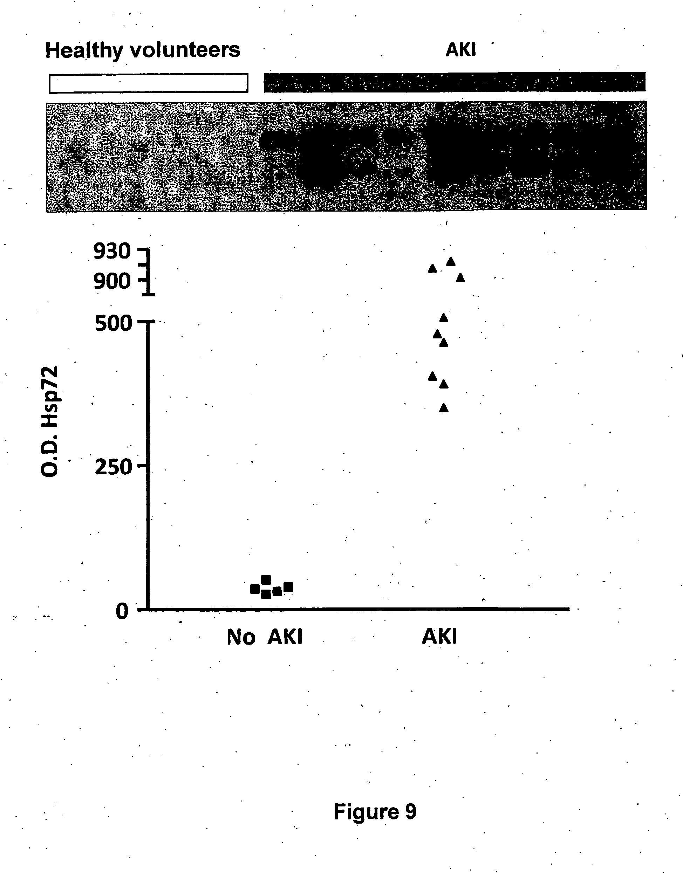

To determine if Hsp72 is a sensitive biomarker for AKI detection in humans, the urinary levels of this protein were analyzed by Western blot in healthy donors and compared with those patients that developed AKI en the intensive care unit. AKI was defined as an increase in serum creatinine of at least 0.3 mg/dL or a urinary volume less than 0.5 ml/kg/h for 6 hours. Table 1 shows the general characteristics and renal function from five healthy kidney donors and 9 patients with septic AKI. In the group of AKI patients, 5 were female and 4 male with an age ranging between 24 and 84 years. At the ICU admission, all of the patients displays normal serum creatinine values, however, creatinine increases from 0.55 +- 0.05 to 2.30 +-0.52 mg/dL during their stay in the ICU, showing the development of AKI. The urinary Hsp72 levels are depicted in the Figure 9. In the urine of healthy kidney donors Hsp72 was almost undetectable (33.7 +- 7.1 arbitrary units), while the Hsp72 levels increased in AKI patients (583 + - 85.1). Of note, two of the patients diagnosed with AKI died during their hospitalization and were from the ones that displayed the higher Hsp72 levels.

**Table 1. General characteristics and renal function in healthy kidney donors and patients with septic acute kidney injury.**

| **Subject Number** | **Gender** | **Age (years)** | **Diagnostics** | **Baseline SCr (mg/dL)** | **SCr at AKI (mg/dL)** | **Follow-up after Sampling (days)** | **Outcome/Last SCr (mg/dL)** |
|---|---|---|---|---|---|---|---|
| **Healthy Kidney Living-Donors** | | | | | | | |
| 1 | | 27 | Healthy | 0.82 | NA | NA | NA |
| 2 | | 25 | Healthy | 0.95 | NA | NA | NA |
| 3 | | 32 | Healthy | 0.85 | NA | NA | NA |
| 4 | | 42 | Healthy | 0.77 | NA | NA | NA |
| 5 | | 35 | Healthy | 0.56 | NA | NA | NA |

| **Septic Acute Kidney Injury patients** | | | | | | | |
|---|---|---|---|---|---|---|---|
| 1 | Female | 51 | Community-Acquired Pneumonia & Morbid Obesity | 0.50 | 1.2 | 122 | Recovery/0.62 |
| 2 | Male | 49 | Abdominal Sepsis | 0.60 | 3.42 | 28 | Death |
| 3 | Male | 24 | Community-Acquired Pneumonia & Systemic Lupus | 0.32 | 0.83 | 24 | Unrecovery/2.42 |
| 4 | Female | 39 | Community-acquired pneumonia & Meningitis & Septic shock | 0.49 | 1.19 | 45 | Recovery/0.48 |
| 5 | Female | 84 | Community-Acquired Pneumonia | 0.73 | 1.14 | 1 | Death |
| 6 | Male | 34 | Community-Acquired Pneumonia | 0.75 | 1.86 | 61 | Improvement/1.12 |
| 7 | Female | 84 | Community-Acquired Pneumonia & Heart Failure | 0.93 | 3.11 | 40 | Recovery/1.04 |
| 8 | Female | 35 | Systemic Lupus & Community-Acquired Pneumonia | 0.44 | 2.19 | 5 | Improvement /1.29 |
| 9 | Male | 46 | Septic Arthritis & Diabetes mellitus | 0.72 | 5.75 | 138 | Recovery/0.77 |

### BRIEF DESCRIPTION OF THE FIGURES

**Figure 1****.** Renal function parameters in rats with sham surgery and underwent ischemia of 10, 20, 30, 45 and 60 min and 24 h of reperfusion. AKI was evidenced by an increase in serum creatinine (A), together with a reduction in creatinine clearance (B) and renal blood flow (C), without changes in the mean arterial pressure (D). *p<0.05 vs. sham.
**Figure 2****.** Sub-cortical histology sections with PAS staining from the kidneys of each group. (A-F). Cast number per field count; five fields per rat were quantified (G). Tubular injured area percentage determined by loss of the brush border and polarity, as well as, cell detachment. *p<0.05 vs. sham.
**Figure 3****.** Tubular injury and oxidative stress markers quantification. The urinary excretion of NAG (A), proteins (B) and H₂O₂ (C) were elevated in the groups with I/R versus the sham. *p<0.05 vs. sham.
**Figure 4.** (A) Hsp72 mRNA levels in the cortex of rats subjected to I/R. (B) Western blot analysis from the Hsp72 protein levels in the kidney cortex and its over-expression in rats underwent to different periods of I/R. (C) Urinary Hsp72 concentration. *p<0.05 vs. sham.
**Figure 5.** (A) Positive correlation between the amount of urinary Hsp72 and cast formation. (B) Relationship between the amount of urinary Hsp72 and the % of tubular affected area with an r= 0.79 and p<0.0001.
**Figure 6.** (A) Western blot analysis form the urinary hsp72 concentration in rats underwent to bilateral ischemia. (B) Correlation between the amount of Hsp72 detected by western blot and cast formation.
**Figure 7.** (A) Agarose gel electrophoresis showing the integrity of the urine extracted RNA. (B). Hsp72 mRNA levels in the urine of rats subjected to I/R. *p<0.01 vs. sham. (C) Correlation between the urinary Hsp72 mRNA levels and cast formation.
**Figure 8****.** Urinary NAG, protein and Hsp72 quantification in rats underwent to 30 min of bilateral ischemia and different periods of reperfusion: 3, 6, 9, 12, 18, 24, 48, 72, 96, 120 h. *p<0.05 vs. sham.
**Figure 9****.** Urinary Hsp72 levels in healthy kidney donors and AKI patients determined by Western blot analysis.

## Claims

1. A method for the early detection of acute kidney injury, comprising the determination of the heat shock protein 72 (Hsp72) concentration as biomarker in a urine sample obtained from a mammal.

2. The method according to claim 1, **characterized in that** said mammal is human.

3. The method according to claim 1, **characterized in that** said Hsp72 in urine sample is detected after three hours following the injury has been provoked in the kidney.

4. The method according to claim 1, **characterized in that** said concentration of heat shock protein 72 allows to stratify the intensity of the injury provoked by increasing periods of acute kidney injury.

5. The method according to claim 1, **characterized in that** the concentration of biomarker is determined at the mRNA level using immunoassays.

6. The method according to claim 1, **characterized in that** the concentration of biomarker is determined at the protein level using immunoassays.

## Patentansprüche

1. Verfahren zur Früherkennung von akuter Nierenläsion, umfassend die Bestimmung der Hitzeschockprotein 72 (Hsp72)-Konzentration als Biomarker in einer Urinprobe von einem Säuger.

2. Verfahren nach Anspruch 1, **dadurch gekennzeichnet, dass** besagter Säuger ein Mensch ist.

3. Verfahren nach Anspruch 1, **dadurch gekennzeichnet, dass** das Hsp72 in der Urinprobe innerhalb von drei Stunden, nachdem die Verletzung in der Niere hervorgerufen wurde, erkannt wird.

4. Verfahren nach Anspruch 1, **dadurch gekennzeichnet, dass** besagte Hitzeschockprotein 72-Konzentration eine Stratifizierung der Intensität der Verletzung, hervorgerufen durch zunehmende Perioden von akuter Nierenläsion, ermöglicht.

5. Verfahren nach Anspruch 1, **dadurch gekennzeichnet, dass** die Biomarker-Konzentration auf der mRNA-Ebene mittels Immunoassays bestimmt wird.

6. Verfahren nach Anspruch 1, **dadurch gekennzeichnet, dass** die Biomarker-Konzentration auf der Protein-Ebene mittels Immunoassays bestimmt wird.

## Revendications

1. Procédé pour la détection précoce de lésion rénale aiguë, comprenant la détermination de la concentration de la protéine de choc thermique 72 (Hsp72) en tant que biomarqueur dans un échantillon d'urine obtenu d'un mammifère.

2. Procédé selon la revendication 1, **caractérisé en ce que** ledit mammifère est humain.

3. Procédé selon la revendication 1, **caractérisé en ce que** ladite Hsp72 dans échantillon d'urine est détectée trois heures après que la lésion a été occasionnée dans le rein.

4. Procédé selon la revendication 1, **caractérisé en ce que** ladite concentration de la protéine de choc thermique 72 permet de stratifier l'intensité de la lésion occasionnée par des périodes croissantes de lésion rénale aiguë.

5. Procédé selon la revendication 1, **caractérisé en ce que** la concentration de biomarqueur est déterminée au taux d'ARNm en utilisant des immunoessais.

6. Procédé selon la revendication 1, **caractérisé en ce que** la concentration de biomarqueur est déterminée au taux de protéine en utilisant des immunoessais.
